# EUROPEAN PATENT APPLICATION

(11) **EP 4 085 896 A1**
(43) Date of publication of application: **09.11.2022**
(21) Application number: 21172110.5
(22) Date of filing: 04.05.2021
(51) Int. Cl.: A61K 9/00, A61K 47/36

(54) **PHARMACEUTICAL ANGIOTENSIN-(1-7) COMPOSITIONS IN THE TREATMENT OF (SARS)-COV- OR (SARS)-COV-2-INFECTION RELATED DISEASES**

(71) Applicant: CU-Pharmaceuticals UG, 44263 Dortmund (DE)
(72) Inventor: SCHMITZ, Uwe, 32756 Detmold (DE)
(74) Representative: Michalski Hüttermann & Partner Patentanwälte mbB

(57) **Abstract**

The invention at hand concerns a pharmaceutical composition comprising Angiotensin-(1-7) (Asp-Arg-Val-Tyr-Ile-His-Pro) for use in the treatment of coronavirus infection related diseases, wherein Angiotensin-(1-7) is administered in a formulation selected from the group consisting of intramuscular, transdermal, pulmonary, lingual, buccal, nasal or a combination of at least two members of said list. Furthermore, the invention relates to a pharmaceutical composition comprising Angiotensin-(1-7) in a pharmaceutical acceptable carrier.

## Description

The invention at hand concerns a pharmaceutical composition comprising Angiotensin-(1-7) (Asp-Arg-Val-Tyr-Ile-His-Pro) for use in the treatment of coronavirus infection related diseases. Angiotensin-(1-7) is administered in a formulation selected from the group consisting of intramuscular, transdermal, pulmonary, lingual, buccal, nasal or a combination of at least two members of said list. Furthermore, the invention relates to a pharmaceutical composition comprising Angiotensin-(1-7) in a pharmaceutical acceptable carrier.

Severe acute respiratory syndrome coronavirus 2 (SARS-CoV-2) is the third coronavirus (CoV)-variant gaining significant attention, essentially based on the infectivity and mortality rates. Previous emerged CoV-variants already showed the potential of threatening diseases, nevertheless, for some reasons SARS-CoV-1 and the Middle-East respiratory syndrome (MERS)-CoV did not lead to a major worldwide pandemic outbreak. The overall physiopathol-ogy of SARS-CoV-2 infections remains until now not fully resolved, albeit a large amount of scientific knowledge and data are present for (SARS)-CoV or (SARS)-CoV-2 related infection. In general, it is accepted in the scientific community that SARS-CoV-2 shares functional similarities compared to SARS-CoV-1, especially the mechanism to enter host cells, by using the receptor angiotensin conversion enzyme 2 (ACE2). Both CoV-1 and CoV-2 infections may result in a complex respiratory failure, clinically known as acute respiratory distress syndrome (ARDS). ARDS is characterized by a rapid onset of widespread inflammation in the lungs. Typical symptoms for instance include shortness of breath (dyspnea) and rapid breathing (tachypnea). Anyhow, (SARS)-CoV or (SARS)-CoV-2 related infections are damaging several specific tissues on a cell level, impair the lungs' ability to exchange oxygen and carbon dioxide, and, uncontrollably, activate the immune response and result in dysfunction of blood clotting.

The severity and the multi-factor impact of a (SARS)-CoV- or (SARS)-CoV-2-infection on the human body can, at least in part, be explained by a side-effect of the infection. As indicated above, SARS-CoV-2 utilizes a specific enzyme of the renin-angiotensin-System (RAS), i.e. ACE2, for cell entry. Upon binding to CoV, ACE2 may be removed from the cell membrane, thereby reducing ACE2 on the cell surface and increasing ACE2 in the blood stream. This may lead to overstimulation of the RAS classic pathway with adverse cardiovascular and respiratory effects, resulting in hypercoagulation, endothelial dysfunction, inflammation and insulin resistance.

Despite the already existing treatment options and pharmaceutical formulations in the field of SARS-CoV infections, there is still an interest in new treatments and pharmaceutical compositions, wherein said compositions are able to improve the health status of COVID- and especially of COVID-19-patients.

This task is fulfilled by a pharmaceutical composition comprising Angiotensin-(1-7) for use in the treatment of COVID, especially (SARS)-CoV or (SARS)-CoV-2 infection related diseases and a pharmaceutical composition comprising Angiotensin-(1-7) as defined in the independent claims. Preferred embodiments thereof are set forth in the dependent claims.

Above mentioned task is achieved by a pharmaceutical composition comprising Angiotensin-(1-7) (Asp-Arg-Val-Tyr-Ile-His-Pro) for use in the treatment of coronavirus infection related diseases, wherein Angiotensin-(1-7) is administered in a formulation selected from the group consisting of intramuscular, transdermal, pulmonary, lingual, buccal, nasal or a combination of at least two members of said list.

Surprisingly, it was found that a pharmaceutical composition comprising Angiotensin-(1-7) is able to reduce the severity of a coronavirus infection like (SARS)-CoV- or (SARS)-CoV-2-infections by administration of a formulation selected from above-described group of formulations. The formulations can be used to administer target specific and reproducible a predefined amount of Angiotensin-(1-7). The Angiotensin-(1-7) is storage stable within the formulations and, based on the chosen vehicles, the availability of the drug-peptide and the release from the formulation can be tailored with respect to the desired location. This results in an overall improved bioavailability of the drug and, consequently, the clinical symptoms of a (SARS)-CoV- or (SARS)-CoV-2-infection can be reduced in a relatively short treatment time. The administration of Angiotensin-(1-7) is able to improve the overall health status of the patient, tackling several different clinical symptoms at once, because the Angiotensin-(1-7) is, at least in part, also acting on the overall disbalanced RAS-system. Such outcome is surprising, because Angiotensin-(1-7) represents a chain segment rather at the end of the RAS-cascade and most of the research work is tackling the impact and the influence of the first members of the RAS like, for instance, ACE or ACE2. In addition, in certain cases it is possible to achieve a depot-effect, wherein the peptide is released over an increased amount of time, resulting in a sustained release effect including more constant Angiotensin-(1-7) levels in the blood or in the tissue. The formulations are based on non-toxic ingredients and show an improved biocompatibility, resulting in an administration without or with very low incidences of unwanted side-effects. Based on the efficacy of the drug a variety of different symptoms of (SARS)-CoV- or (SARS)-CoV-2-infections can be treated in all phases of the infection, e.g. at the beginning comprising low overall virus loads or even in late stages, wherein hospitalization or even intensive care including ventilation of the infected person is necessary.

The inventive use concerns a pharmaceutical composition comprising Angiotensin-(1-7) (Asp-Arg-Val-Tyr-Ile-His-Pro). Angiotensin-(1-7) as such (H-Asp-Arg-Val-Tyr-Ile-His-Pro-OH, C₄₁H₆₂N₁₂O₁₁) comprises a molecular weight of approx. 899 g/mol is an active heptapeptide of the RAS. This peptide can be used within the inventive formulations as such or as a derivative, an analogue, in a salt, solvated or in an amorphous or ordered crystalline form. An Angiotensin-(1-7) derivative is a molecule comprising the same amino-acid sequence but the amino-acid side-chains may comprise different functional moieties compared to the formula given above . An Angiotensin-(1-7) analogue may for instance comprise additional intramolecular covalent bonds between different amino-acids. A typical amino acid distribution-ratio of the Angiotensin-(1-7) for the single amino-acids may range from Asp: 0.85 to 1.15; Pro: 0.85 to 1.15; Val: 0.80 to 1.20; Ile: 0.80 to 1.20; Tyr: 0.85 to 1.15; His: 0.80 to 1.20; Arg: 0.85 to 1.15. The monoisotopic mass may range from 897.5 up to 899.5 Da. The Angiotensin-(1-7) may not only be administered as such. The Angiotensin-(1-7) is administered in the form of a pharmaceutical composition, at least comprising a further constituent. This constituent can be a further chemical substance either known as excipient or a further compound which can be regarded part of the drug substance, for instance a counter ion or co-solvent.

The Angiotensin-(1-7) is used in the treatment of coronavirus infection related diseases, for instance (SARS)-CoV, like for instance (SARS)-CoV-1 or (SARS)-CoV-2 and coronavirus variants showing a similar mode of action and severity, like MERS. Angiotensin-(1-7) can be used in order to reduce the severity of the impact of a (SARS)-CoV- or (SARS)-CoV-2-infection on the human body. The diseases may originate from a more or less early stage in the infection, for instance including common cold-like symptoms or may originate from heavy infections in the late stage. To the latter group of diseases or symptoms acute lung injury (ALI) and in its most severe form, ARDS as defined by the Berlin criteria (bilateral shadowing on lung radiology, rapid deterioration of symptoms, and objective hypoxemia on blood samples) can be mentioned. The manifestation of these diseases may be classified, for instance, as diffuse alveolo-capillary injury, neutrophilic alveolitis and an increased lung permeability associated with a strong inflammatory response, hypoxic pulmonary vasoconstriction, distal thrombosis, severe hypoxemia and/or a proteinaceous lung oedema.

Coronaviridae is a family of enveloped, positive-strand RNA viruses including the subfamilies Letovirinae and Orthocoronavirinae; the members of the latter are known as coronaviruses.

The viral genome of the Coronaviridae is approximately 26-32 kilobases in length and the virus particles are typically decorated with large (~20 nm), club- or petal-shaped surface projections (the "peplomers" or "spikes"), which in electron micrographs of spherical particles create an image resembling the solar corona. The 5' and 3' ends of the genome have a cap and poly(A) tract, respectively. The viral envelope, obtained by budding through membranes of the endo-plasmic reticulum (ER) or Golgi apparatus, invariably contains two virus-specified (glyco)protein species, S and M. Glycoprotein S comprises the large surface projections, while M is a triple-spanning transmembrane protein. Another important structural protein is the phospho-protein N, which is responsible for the helical symmetry of the nucleocapsid that encloses the genomic RNA

Angiotensin-(1-7) can be administered in a formulation adjusted for intramuscular injection. Intramuscular injection (iM), is the injection of a substance into a muscle. It is one of several methods for parenteral administration of drugs. Intramuscular injection may result in a faster drug absorption compared to subcutaneous or transdermal injections and may provide the advantage that the drug is not subject to a first-pass metabolism effect affecting oral medications. Common sites for intramuscular injections include for instance the deltoid muscle of the upper arm and the gluteal muscle of the buttock.

Angiotensin-(1-7) can be administered in a formulation adjusted for transdermal application. Transdermal applications include the delivery of drugs across the skin for systemic distribution in the human body. Examples include for instance transdermal patches or ointments that delivers the drug firstly into the dermal tissues and secondly in the blood stream for achieving a systemic effect.

Angiotensin-(1-7) can be administered in a formulation adjusted for pulmonary application. Angiotensin-(1-7) can also be administered via the respiratory epithelial cells. Therefore, the pulmonary route can be used as a non-invasive administration for systemic and local delivery of Angiotensin-(1-7), based on the high permeability and large absorptive surface area of the lungs. Especially, the alveolar epithelium of the distal lung has been shown to be an absorption site for Angiotensin-(1-7). Further advantages over peroral applications are the comparatively low enzymatic activity, rapid absorption of Angiotensin-(1-7) and the capacity for overcoming first-pass metabolism.

Angiotensin-(1-7) can be administered in a formulation adjusted for lingual application. Lingual applications include lingual and sublingual application. In this delivery system Angiotensin-(1-7) is absorbed via the mucous including and surrounding the tongue. Angiotensin-(1-7) diffuse into the blood through the mucous gland that produces a thick mucinous fluid and lubricates the oral cavity which allows for swallowing, initiating digestion, buffering pH, and dental hygiene. The sublingual glands receive their primary blood supply from the sublingual and submental arteries, which are branches of the lingual artery and facial artery, respectively. These arteries are both branches of the external carotid artery. The sublingual vein drains into the lingual vein, which then flows into the internal jugular system. Such route can lead to a systemic distribution of Angiotensin-(1-7).

Angiotensin-(1-7) can be administered in a formulation adjusted for buccal application. Buccal administration is a topical route of administration by which Angiotensin-(1-7) held or applied in the buccal diffuse through the oral mucosa and enters directly into the bloodstream. Buccal administration may provide better bioavailability of some drugs and a more rapid onset of action compared to oral administration because the medication does not pass through the digestive system and thereby avoids first pass metabolism.

Angiotensin-(1-7) can be administered in a formulation adjusted for nasal application. Nasal administration is a route of administration in which drugs are insufflated through the nose. Angiotensin-(1-7) can be administered by this route systemically. Possible formulations may include nasal sprays are nasal ointments.

Angiotensin-(1-7) can be administered at the same time using two different formulations including a combination of above-described formulation types. In order to effectively treat the CoV induced diseases it is possible to use a combination treatment, wherein at the same time interval two different delivery system are used. For instance, it is possible to administer the Angiotensin-(1-7) by iV and, in addition, via a nasal spray. Based on the two different delivery systems a different bioavailability in different body tissues can be achieved at the same time.

Within a preferred embodiment of the use the formulation can be a transdermal formulation and the transdermal formulation comprises Angiotensin-(1-7) in a form selected from the group consisting of oil-in-water, water-in-oil, gel, solid-lipid nanoparticles (SLN), nanostructured lipid carriers (NLC), microemulsions, transfersomes or a combination of at least two members of said list. Especially, transdermal formulations may be suitable to administer a sufficient amount of Angiotensin-(1-7) in order to reduce the effects of diseases caused by a (SARS)-CoV- or (SARS)-CoV-2-infection.

In a preferred embodiment the transdermal formulation can be a Solid Lipid Nanoparticle (SLN), wherein the SLN consist of a solid lipid matrix comprising a melting point above 40°C. In this particle approximately an Angiotensin-(1-7) concentration of 0.1 - 30% (w/w) is incorporated. Besides SLNs also NLCs, consisting of solid and liquid lipids, comprising approximately a total lipid content of 5 - 40% (w/w) can be used. The NLCs can be stabilized with a surfactant or a surfactant mix. SLN and NLC can be produced including an Angiotensin-(1-7) component according to a number of production techniques listed below. The following excipients can for instance be used in Angiotensin-(1-7) NLC and SLN production:
Solid lipids Beeswax Natural wax with GRAS status, composed of a mixture of fatty acids and fatty alcohols esters, with melting point of 62-64°C; Carnauba wax Natural wax with GRAS status high melting point of 82-85°C, composed of monoesters; Cetyl palmitate Synthetic wax produced by esterification of cetyl alcohol and palmitic acid, with melting point between 40.5-51°C; Compritol^{®} 888 ATO Blend of different esters of behenic acid with glycerol; it holds acceptable safety profile, with a melting point of 69-74°C; Dynasan^{®} Triglycerides series from Sasol; a group of natural and safe lipids, which includes Dynasan 112 (trilaurin; melting point 46°C), Dynasan 114 (trimyristin; melting point 55-58°C), Dynasan 116 (tripalmitin; melting point 61-65°C), and Dynasan 118 (tristearin; melting point 70-73°C); Gelucire^{®} Series of lipid from Gatefossé defined by their melting points between 33-70°C and by the HLB between 1 - 18; Gelucire 50/13 (stearoyl macrogol-32 glycerides); Precirol^{®} ATO 5 Glyceryl palmitos-tearate, is a mixture of mono, di and triglycerides of palmitic and stearic acid, melting point of 58°C; Softisan^{®} 378 Blend of triglycerides with hydrocarbon-chain length of C8-C18, low melting point of 35-42°C; Stearic acid Endogenous long-chain saturated fatty acid, with melting point around 70°C and HLB around 15; Liquid lipids Miglyol^{®} 812 Triglycerides of capric and caprylic acid, are medium chain triglycerides with high stability against oxidation, high solubility for many drugs; oleic acid pure substance used as emulsifying agent and penetration enhancer; squalene triterpene produced by human skin cells; vitamin E/α -tocopherol liquid lipid with the advantage of providing protection to oxidation sensitive substances; surfactants Lecithin, with HLB between 4-9; Plantacare^{®} 810 caprylyl/capryl glucoside is a highly effective stabilizer for SLN and NLC, with HLB 15 - 16; poloxamer^{®} 188 nonionic triblock copolymer with hydrophilic and lipophilic units, used as emulsifier and stabilizing agent, with HLB > 24; Quillaja saponin Natural saponin-based surfactant, isolated from the tree Quillaja saponaria , composed by a complex mixture of amphiphilic constituents; sodium lauryl sulfate and sodium dodecylsulphate is an anionic surfactant, HLB ≈ 40; Tween^{®}80 Polyoxyethylene sorbitan monooleate, or polysorbate 80, is an O/W surfactant HLB around 15.

The SLN and NLC can for instance be produced by Hot High-Pressure Homogenization (HHPH). In this process Angiotensin-(1-7) is dissolved in the melted lipid phase and mixed with the aqueous emulsifier phase at high temperature (5-10°C above melting temperature) using a high-speed stirrer (e.g., Ultra Turrax, Pre-Formulation). The formulation is afterwards transferred to a high-pressure homogenisator. The numbers of homogenisation cycles depend on the formulation itself and cab be directly proportional to the lipid mass.

The SLN and NLC can for instance be produced by Cold High-Pressure Homogenisation (CHPH). In this process Angiotensin-(1-7) is dissolved in a melted lipid phase. After dissolving the phase is rapidly cooled using dry ice or nitrogen. The solid phase is then chopped and rapidly mixed with the cold aqueous surfactant solution. The dispersion is added to the homogenizer and treated for a low number (<5) of cycles at room temperature or below RT.

The SLN and NLC can for instance be produced by a microemulsion technique. In this process Angiotensin-(1-7) is dissolved and the lipid and aqueous phase including the surfactant is prewarmed to the same temperature and homogenized under mild stirring. The emulsion is dispersed in cold water (2-10°C) under stirring and washed using distilled water.

The SLN and NLC can for instance be produced by emulsification solvent diffusion. In this process Angiotensin-(1-7) is incorporated in a O/W-emulsion, wherein Angiotensin-(1-7) is solved in the organic phase. The organic phase is miscible with water. After dissolving both phases and evaporation of the organic phase drug nanoparticles are formed.

The SLN and NLC including Angiotensin-(1-7) can for instance be produced by a membrane contactor technique. This method uses fine porous membranes (pores sizes < 0.05 µm) where the molten lipid phase is filtered into the aqueous phase.

The Angiotensin-(1-7) SLN and NLC can for instance be produced as transfersomes embedded in a gel. For producing Angiotensin-(1-7) transfersomes the lipids soja lecithin and cholesterol are transferred to a clean beaker. Tween 80 is dissolved in a diethyl ether chloroform mixture in the same beaker and stored for 24 h at room temperature. The Angiotensin-(1-7) is dissolved in the aqueous phase and transferred to the lipid film and sonication 20 kHz for 2 min. After that the film is rehydrated in PBS buffer and sonicated. For instance 2% (v/v) DMSO can be added and the transfersomes are passed through a filter paper. Transfersomes can be transferred to a 5% (w/v) methylcellulose gel.

Further methods for administration of Angiotensin-(1-7) via the dermal route may additionally include physical enhancement techniques such as iontophoresis, electroporation, sonophoresis, laser ablation, thermal ablation, microneedles etc.. Such physical enhancement techniques may be able to further increase the Angiotensin-(1-7) administration via the dermal route.

In a further preferred aspect of the use the formulation is a pulmonary formulation and the pulmonary formulation comprises Angiotensin-(1-7) in a form selected from the group consisting of liposomal encapsulated, liquid suspension, dry powder or a combination of at least two members of said list. Especially, pulmonary formulations may be suitable to administer a sufficient amount of Angiotensin-(1-7) in order to reduce the effects of diseases caused by a CoV-infection, e.g. (SARS)-CoV or (SARS)-CoV-2. Angiotensin-(1-7) can be administered via a pulmonary formulation including an inhalation step. The advantage of this route is that no safety issues were encountered. High Angiotensin-(1-7) amounts can be administered via such route, e.g. 10 mg/mL of Angiotensin-(1-7) acetate in a PBS-buffer.

Possible further ingredients compatible with an Angiotensin-(1-7) drug in pulmonary formulations may include: 1,2-distearoyl-sn-glycero-3-phosphocholine, alcohol, anhydrous citric acid, anhydrous trisodium citrate, apaflurane, ascorbic acid, benzalkonium chloride, black ink, black ink, calcium carbonate, calcium chloride, carrageenan, cetylpyridinium chloride, cetylpyridinium chloride, chlorobutanol, chlorobutanol, citric acid monohydrate, dichlorodifluoromethane, dichlorotetrafluoroethane, edetate disodium, ferric oxide yellow, fluorochloro-hydrocarbons, gelatin, glycerin, glycine, hydrochloric acid, hypromellose 2906 (4 mPas), lactose, lactose monohydrate, lecithin, soybean, magnesium stearate, mannitol, menthol, menthol, methylparaben, nitric acid, norflurane, n-phenyl-1-napthylamine, nutmeg oil, oleic acid, petrolatum, phenylethyl alcohol, polysorbate 80, potassium chloride, propylene glycol, propylparaben, saccharin, saccharin, saccharin sodium, silicon dioxide, sodium bicarbonate, sodium bisulfate, sodium bisulfite, sodium chloride, sodium hydroxide, sodium lauryl sulfate, sodium metabisulfite, sodium sulfate anhydrous, sorbitan trioleate, sorbitan trioleate, sulfuric acid, thymol, titanium dioxide, trichloromonofluoromethane, trisodium citrate dihydrate, tromethamine, turpentine oil, zinc oxide.

Possible formulations for an efficient pulmonal administration route for Angiotensin-(1-7) may include:
- Metered dose inhaler (MDI) based on a liposomal suspension liquid, PulmoSpheres^{®};
- Dry powder inhaler (DPI), based on spray dried liposomes, Angiotensin-(1-7) powder, PulmoSpheres^{®};
- Nebulizer, an Angiotensin-(1-7) suspension in a liquid form, PulmoSpheres^{®}.

A suitable liposomal formulation for pulmonary delivery including Angiotensin-(1-7) for MDI or DPI can include the following processing. For this formulation type, various lipids like 1,2-Dipalmitoyl-sn-glycero-3-phosphocholine (DPPC) or 1,2-Dioleoyl-sn-glycero-3-phosphocholine (DOPC), mixed with cholesterol were diluted in organic solvent (e.g. ethanol). The desired Angiotensin-(1-7) solution and the lipid solution are loaded on the microfluid device (e.g. precision nanosystems). The liposomes are formed through a self-assembling process in the narrow channels of the chip, which can contain the desired Angiotensin-(1-7) amount as a deposit in the inner aqueous phase. If necessary, other production methods can be used, for instance a thin-film hydration method or an ethanol-injection method. The liposomal formulation can be inhaled as suspension via metered dose inhaler (MDI) or as a spray dried powder via a dry powder inhaler (DPI).

A possible manufacturing process for a pulmonary delivery including a liposomal encapsulated Angiotensin-(1-7) may include the following: A NanoAssemblr from Precision NanoSystems can be used. The production technique is based on a mixing of an aqueous phase including Angiotensin-(1-7) and an organic phase, which contain dissolved lipids. This self-assembling process leads to liposome formation. To regulate the supply of the two phases, the connection to a pump system is necessary. A Flowrate of 12 ml/min and a tube size of 1.44 mm can for instance be used. Liposomes can be collected in a suitable container. The liposome production may be based on an organic phase comprising: DPPC/Ph 90H/DOPC to cholesterol ratio of 7: 3, total amount 200 mg in 5 ml EtOH (99.8%), Stirrer 50°C for 20 min; The aqueous phase may include 0.5-50 µg of Angiotensin-(1-7) and HPβCD in saline. Alternatively, the aqueous phase may include 0.5-50 µg/mL Angiotensin-(1-7) in 5 mL saline/DPBS; alternatively, 0.5-50 µg/mL Angiotensin-(1-7) + various excipients (e.g. citric acid, ascorbic acid, see table supplementary) to modify e.g. the pH and the Angiotensin-(1-7) release profile.

The liposomal encapsulated Angiotensin-(1-7) may be purified by centrifugation at larger than 4000 g for 20 min at 4°C and resuspension, e.g. in pre-cooled DPBS at 4°C.

A possible manufacturing process for pulmonary delivery may include a liquid (suspension) formulation for MDI based on Angiotensin-(1-7) formulated as liquid, e.g. in the form of a suspension or solution. The latter can be applied via MDI. Therefore, following components can be used: Angiotensin-(1-7) in a concentration of 0.5-50 µg/mL + various excipients (e.g. citric acid, ascorbic acid) for adapting e.g. the pH.

In addition, or alternatively PulmoSpheres can be used for an Angiotensin-(1-7) pulmonary delivery system. These particles can be produced by using an emulsion-based spray-drying process. In a first step, oil-in-water emulsion droplets are produced by high-pressure homogenization with the oil phase perfluorooctyl bromide (PFOB). Afterwards, a spray-drying process leads to phospholipid-based small, porous particles. The advantages of these particles are the optimized lung targeting and a consistency of the dose. These particles can be solubilized in the desired solvent.

A possible route of processing Angiotensin-(1-7) pulmospheres may include calcium chloride as surface modifier; phospholipids, preferably long-chain phospholipids, e.g. distearoylphosphatidylcholine as emulsifier; water and perfluorooctyl bromide as solvents. The solution can be based on 0.5 - 50 µg/mL of Angiotensin-(1-7) dissolved in the oil-in water emulsion. The suspension may comprise > 1 mg/mL Angiotensin-(1-7) as fine particles. A possible composition may include DSPC (70 mg/ml), cholesterol (30 mg/ml), 150 mM NaCl, 30 mM CaCl₂ buffer (pH 7.0, or CaCl2 (0-100 mg/100 g aqueous phase) + 0.5 - 50 µg/mL Angiotensin-(1-7) in 5 mL water (or PBS, saline) and 5 mL PFOB solution.

A further pulmonary delivery system for Angiotensin-(1-7) may be an inhalable powder, wherein in the powder micronized Angiotensin-(1-7) particles are included for the generation of aerosols by DPI. Suitable Angiotensin-(1-7) excipients may include carbohydrates like trehalose, lactose, and/or mannitol. Besides using a spray dried powder also Angiotensin-(1-7) nanoparticles can be used. These solid Angiotensin-(1-7) nanoparticles can for instance be formed by spray drying.

It is also possible to use Angiotensin-(1-7) in a nano- in microparticle approach. The nanoparticles are loaded into a carrier microparticle to improve the handling and delivery of the Angiotensin-(1-7) into the deep lung. It is possible to use SIMANIM particles, i.e. lactose, poly(lactide-co-glycolide) (PLGA) and dipalmitoylphosphatidylcholine (DPPC). A possible route of processing SIMANIM particles may include: PLGA and DPPC are dissolved in dichloromethane, homogenisation with an aqueous solution of 0.5 - 50 µg/mL Ang 1-7 in PBS/saline; Homogenisation in an aqueous lactose solution, followed by spray drying.

An alternative form of Angiotensin-(1-7) may be a nanoaggregate dispersion with a polymer coated surface. In order to increase the Angiotensin-(1-7) mucopenetration or opsonin defence, polymers can be used to coat nanoparticles (ethylene glycol) (PEG), methoxy poly(ethylene glycol) (MPEG), 1,2-dilauroyl-sn- glycero-3-phosphocholine (DLPC) and vitamin E d-a-succinated polyethylene glycol 1000 (vitamin E TPGS). The production of Angiotensin-(1-7) PEG-PLGA- particles may include: Step 1: 120 mg PLGA will be solved in 6 mL ethyl acetate (small beaker on a stirring plate) for approximately 30 minutes (maximum 2 hours). 0.5 - 50 µg/mL Ang 1-7 are mixed in the organic phase. Step 2: 5 mL PLGA-API solution is added dropwise (syringe pump) to an organic solvent solution (acetone, ethyl acetate etc.) under stirring. Step 3: The particles were incubated in a PEG, e.g. a PEG6000 solution for 4 h under stirring in order to coat the particles. Step 4: Spray drying.

Angiotensin-(1-7) can also be used in combination with a nebulizer for pulmonary delivery. Suitable further excipients may be selected from the group of sodium chloride, dextrose, hydrochloric/sulfuric acid, nitrogen , sodium citrate, sodium phosphate, citric acid, polysorbate 20, 80, disodium EDTA, CMC, Na-CMC, alcohol, PEG 400, propylene glycol, glycerin and combinations of at least two members from said list.

In another preferred characteristic of the use the formulation can be a lingual/buccal formulation and the lingual/buccal formulation comprises Angiotensin-(1-7) in a form selected from the group consisting of chewing gum, sublingual, dry powder or a combination of at least two members of said list. Especially, lingual/buccal formulations may be suitable to administer a sufficient Angiotensin-(1-7) amount in order to reduce the effects of diseases caused by a CoV, especially (SARS)-CoV- or (SARS)-CoV-2-infection. For a suitable administration of Angiotensin-(1-7) via the lingual/buccal route an encapsulation of angiotensin into chewing gum or an encapsulation into shear sensitive polymer/hydrogels is possible and advantageous.

The preparation of shear sensitive hydrogels including Angiotensin-(1-7) can be based on polyethylene glycol (PEG). Various PEG-variants can be gelled by the use of acids. One example for an acid can be citric acid. Both substances qualify for a buccal formulation. Additionally, PEG can be gelled together with cellulose derivatives (e.g. carboxymethylcellulose) to obtain gels with special properties such as thermoresponsive properties. The Angiotensin-(1-7) has to be added to the hydrogel during preparation. Upon gelation the angiotensin is entrapped into the gel structure and released on chewing.

A possible preparation of Angiotensin-(1-7) in a chewing gum may include chicle as a base substance. Additionally, flavours, sweetener and citric acid can be added. Angiotensin-(1-7) can be incorporated in a hydrogel and can be added during to the liquid chewing gum preparation during production. Alternatively, Angiotensin-(1-7) can be encapsulated into mucoadhesive nanoparticles, e.g. based on chitosan. The particles stick to mucosa and release Angiotensin-(1-7) over time. The particles can be prepared as micro-, submicron- or nanoparticles. The preparation can be conducted by single or double emulsion solvent diffusion (evaporation), precipitation, coagulation or spray drying techniques. It is also possible to use a multiple encapsulation technique, e.g. Angiotensin-(1-7) encapsulated in nanoparticles that are encapsulated into microparticles.

Several polymers can serve as particle materials. Generally, all polymers that are available for oral drug delivery can be considered appropriate for being used with Angiotensin-(1-7). This includes for example: PLGAs, PLAs, PGAs, PEG-PLGAs, pNIPAm, methacrylate polymers (e.g. Eudragit), poly-sugar based polymers like for example chitosan, alginate, starch, gluco- and galactomannans, dextrans and maltodextrins. Especially substances that have a mucoadhesive property are interesting for preparation of such particles and can be attached to the buccal mucosa and release Angiotensin-(1-7) continuously for an extended period of time.

A possible route of preparation of a buccal/lingual dosage form may include:
1. Particle preparation: precipitation setup (nanoparticles/submicron particles)
   a. Dissolve PLGA in ethyl acetate
   b. Dissolve Angiotensin-(1-7) in the same batch of ethyl acetate
   c. Dissolve chitosan in water
   d. If Angiotensin-(1-7) is insufficiently soluble in ethyl acetate, dissolve it in the same water as the chitosan
   e. Add stabilizer to the chitosan solution (e.g. pluronics, tweens or PVA)
   f. Take prepared PLGA-solution into a syringe with a needle
   g. Inject PLGA-solution into stirred Chitosan solution (PLGA forms nanoparticles by precipitation including Angiotensin-(1-7) and chitosan into them.
2. Particle preparation: spray drying setup (microparticles)
   a. Dissolve chitosan in water
   b. Add Angiotensin-(1-7) to water (also as particles prepared in part 1 possible)
   c. Add stabilizer
   d. Conduct spray drying process
3. Chewing gum preparation
   a. Heat the chewing gum base mass (e.g. chicle) until it is liquified and mix it with sugar, citric acid, flavor and additional components
   b. Stir substance into a homogeneous mass
   c. Upon cooling before hardening add the particles solution and knead it into the chewing gum mass until homogeneity is achieved.

Within a preferred embodiment of the use the formulation can be a nasal formulation and the nasal formulation comprises Angiotensin-(1-7) in a form selected from the group consisting of nanoparticles, oil-in-water, water-in-oil, gel, hyaluronic acid based aqueous solution, DMSO-based solution, dry powder or a combination of at least two members of said list. Especially, nasal formulations may be suitable to administer a sufficient amount of Angiotensin-(1-7) in order to reduce the effects of diseases caused by a CoV, especially a (SARS)-CoV- or (SARS)-CoV-2-infection.

Suitable Angiotensin-(1-7) nasal formulations based on PLGA-Chitosan nanoparticles may comprise one or more of the following characteristics: The Angiotensin-(1-7) can be present as an Angiotensin-(1-7) loaded PLGA-Chitosan nanoparticle. The application is in the form of a nasal spray. The pH can be in the range form 4.3-7.8, preferably: 5.5-6.8. The osmolarity can be adjusted by e.g. NaCl to <500 mOsm/kg, preferably ~280-300 mOsm/kg. The particle size can be < 200 nm. The particle zetapotential can be > 15mV and the particle droplet size can preferably be larger than 30 and smaller than 120 µm. Such a formulation comprises the advantages of a mucoadhesive formulation for nasal delivery, improved bioavailability due to positively charged nanoparticles and the formulation is biodegradable.

A suitable route of preparation may include: Poly(D,L-lactide-co-glycolide) (PLGA) based nanoparticles coated with Chitosan, which can be prepared via an emulsion-diffusion-evaporation technique. In a two-step procedure the aqueous phase is prepared:
Step 1: Polyvinyl alcohol is dissolved in ultra-pure water (e.g. at 2.5 % (w/v)) over night at 50°C (stirred conditions) in a glass container. The system can be closed to avoid evaporation. After the PVA is completely dissolved, the PVA solution is filtered by a vacuum filtration (e.g. using a 0.2 µm filter). The solution can be stored in aliquots at 4°C for several weeks.

Step 2: Chitosan is dissolved for approximately 3 hours in an aqueous, e.g. 2.5 % (w/v) polyvinyl alcohol (PVOH) solution on a stirring plate. The solution has to be used within the next day. The system will be closed to avoid evaporation.

The preparation of the organic phase is achieved by dissolving PLGA in ethyl acetate by using a small beaker on a stirring plate for approximately 30 minutes. Angiotensin-(1-7) is mixed in the organic phase. The system can be closed to avoid solvent evaporation.

The preparation of the nanoparticles may include: The aqueous PVA solution with dissolved chitosan-HCL is placed on a stirring plate with 1000 rpm in a beaker glass which is protected from evaporation. The organic phase may consist of PLGA in ethyl acetate. The solution is added dropwise to the aqueous solution with chitosan-HCl. This can be done by a syringe pump and glass syringe 5 with needle (e.g. Hamilton ø 0,26 mm; ø 0,41 mm; ø 0,13 mm) at a flow rate of larger than 100 µL/min for instance for a duration of approximately half an hour to create the emulsion.

The emulsion is stirred followed by homogenization (fast transfer from stirrer to homogenizer) using an Ultra turrax. Ultra-pure water is added. The nanoparticle dispersion is stirred overnight to evaporate the ethyl acetate at room temperature. The resulting nanoparticles can be stored as aqueous suspension at least for 1 month at 4-6°C. For a better conservation and a guaranteed sterility of the nanoparticle suspension a filtration step can be performed. The nanoparticles can for instance be stored as aqueous suspension for 3 months at 4-6°C.

The final nasal formulation can be prepared based on a 0.9% NaCl solution, wherein the nanoparticles are added in a concentration of e.g. 2 mg/mL, the solution can be stirred overnight and the pH can be adjusted to pH 5-7.

Alternative also a hyaluronic-acid-Angiotensin-(1-7) nasal spray formulation can be used for nasal delivery of Angiotensin-(1-7).

Possible characteristics of the nasal spray may include one or more of the following aspects: The Angiotensin-(1-7) can be present as an Angiotensin-(1-7) loaded hyaluronic-acid. The application can be in the form of a nasal spray. The pH can be in the range form 4.3-7.8, preferably: 5.5-6.8. The osmolarity can be adjusted e.g. by addition of NaCl to <500 mOsm/kg, preferably approximately 280-300 mOsm/kg. The particle size can be in the range from about 30 µm up to 120 µm . The zetapotential can be > 15mV. Such a formulation may comprise the advantages of a mucoadhesive formulation for nasal delivery, improved bioavailability due to positively charged nanoparticles and a biodegradable formulation.

The final nasal formulation can be prepared based on a 0.9% NaCl solution, wherein hyaluronic acid is added in a concentration of e.g. 2 mg/mL and Angiotensin-(1-7) is added in a concentration ranging from 0.5 - 50 µg/ml. The solution is stirred overnight and the pH may be adjusted to pH 5-7, especially pH 6.0.

Within another alternative of a nasal spray Angiotensin-(1-7) formulation the Angiotensin-(1-7) can be incorporated in a DMSO-solution.

Possible features of the nasal spray may include one or more of the following characteristics: The Angiotensin-(1-7) can be present as an Angiotensin-(1-7) dissolved in DMSO. The application can be in the form of a nasal spray. The pH can be in the range form 4.3-7.8, preferably pH 5.5-6.8. The osmolarity can be adjusted e.g. by NaCl addition to <500 mOsm/kg, preferably approximately 280-300 mOsm/kg. The particle size can be in the range from about 30 µm up to 120 µm . The zetapotential can be > 15mV. DMSO can be incorporated in the formulation up to 5 weight-%. Such a formulation comprises the advantages of an improved bioavailability based on the presence of the permeation enhancer.

The final nasal formulation can be prepared based on a 0.9% NaCl solution, wherein for instance 5 weight-% DMSO is added. Angiotensin-(1-7) can be added in a concentration ranging from 0.5 - 50 µg/ml, the solution can be stirred overnight and the pH can be adjusted to pH 5 - 7, especially pH 6.0.

The above-mentioned delivery systems are able to deliver Angiotensin-(1-7) in a highly effective way either systemically or very specific to the desired tissue. In addition, based on the safety profile these delivery systems can be combined with several state-of-the-art ARDS-treatments like the use of glucocorticoids, ketoconazole, lisofylline, alprostadil, inhaled NO.

In addition, several other treatments like corticosteroids, e.g. methylprednisolone, GS-5734/remdesivir, Chloroquine, Ritonavir + lopinavir (Kaletra), Ribavirin + ritonavir + lopinavir, Darunavir (with/without cobicistat), Emtricitabine + tenofovir, Ruxolitinib, IFN- α 2b (Peglntron^{®}, Sylatron^{®}, IntronA^{®}), Baloxavir marboxil (Xofluza), Favipiravir, Arbidol (Umifenovir), Novaferon, Nova can be used in combination with Angiotensin-(1-7).

Principally it is possible to combine Angiotensin-(1-7) in the described delivery systems with every form of antimalarial drugs, anthelmintic and anti-protozoal drugs, antiviral therapy, antibacterials and/or neutralizing monoclonal antibodies.

Suitable antiparasitic drugs can be selected from the group consisting of Chloroquine, Hydroxychloroquine, Chloroquine monophosphate, Chloroquine diphosphate, Niclosamide, Nitazoxanide or mixtures of at least two components thereof.

Suitable antiviral agents can be selected from the group consisting of Indinavir, Lopinavir, Ritonavir, Atazanavir, Darunavir, Tipranavir, Fosamprenavir, Abacavir, Elvitegravir, Raltegravir, Remdesivir, Favipiravir, Sofosbuvir, Ribavirin or mixtures of at least two components thereof.

Suitable antineoplastic agents can be selected from the group consisting of Carfilzomib, Bortezomib, Imatinib, Carrizumab or mixtures of at least two components thereof.

A suitable antibiotic may be Azithromycin.

Suitable neutralizing monoclonal antibodies can be CR3022 or Meplazumab or mixtures of the two components.

Suitable Immunoglobulins may be specific human gamma globulins or a mixture present in convalescent plasma.

Suitable Interferons can be selected from the group consisting of IFN β, IFN α -2a, IFN α or mixtures of at least two components thereof.

Suitable cytokine storm inhibitors can be selected from the group consisting of Baricitinib, Tocilizumab, Siltuximab, CVL218 or mixtures of at least two components thereof.

Suitable ACE inhibitors (ACEi) can be selected from the group consisting of captopril, enalapril, angiotensin receptor 1 (AT1R) inhibitors, including losartan and valsartan.

It is also possible to combine Angiotensin-(1-7) with soluble human ACE2 (hrsACE2) or a single chain variable fragment (scFv) that binds to the ACE2 receptor to inhibit its complexation with SARS-CoV, especially SARS-CoV-2 and the mutants thereof, respectively.

It is also possible to combine Angiotensin-(1-7) with a serine protease inhibitor like camostat mesylate to further prevent the entry of the virus into host cell by blocking TMPRSS2 activity.

It is also possible to combine Angiotensin-(1-7) with a SARS-CoV PLpro inhibitor including thiopurine compounds, small molecule inhibitors for instance derived from natural products (tanshinones, geranylated flavonoids and diarylheptanoid inhibitors), zinc ions, zinc conjugates and one or more naphthalene inhibitors. It is also possible to further incorporate 6-mercaptopurine, 6-thioguanine, N-ethylmaleimide and mycophenolic acid into the formulation to further act as inhibitors to suppress its proteolytic activity and deubiquitination independently.

Furthermore, it is possible to combine an Angiotensin-(1-7) delivery system as described above with different kinds of vaccines like D NA-based vaccines, inactivated viruses, nonreplicating viral vectors, protein subunits RNA-based vaccines or convalescent patient sera.

In addition, Angiotensin-(1-7) can be combined with drugs targeting the immune system like Mesenchymal stem cells (MSC), UC-MSCs, WJ-MSCs, Losartan, Dexamethasone, Methylprednisolone, MSCs-derived exosomes, Escin, NK cells, IL15-NK cells, NKG2D CAR-NK cells, ACE2 CAR-NK cells,NKG2D-ACE2 CAR-NK cells, CD24Fc, Emapalumab, Recombinant human interferon Alpha-lb, thymosin alpha 1, Recombinant human interferon I, Anakinra, RoActemra, Sarilumab, Tocilizumab, Aviptadil, Naproxen, Fingolimod.

In another preferred embodiment of the use the formulation can comprise Angiotensin-(1-7) in the form of an encapsulated Angiotensin-(1-7) particle. Angiotensin-(1-7) can advantageously be formulated in an encapsulated from, wherein the encapsulated for can suitably be used in one or more of the above-mentioned delivery systems. Suitable excipients e.g. for micro-encapsulation can be selected from the group consisting of gelatin, casein, whey protein, albumin, zein, soy protein, gluten, bees-wax, Carnauba wax, Paraffin or mixtures of at least two components thereof.

Suitable polysaccharides of various origin used in microencapsulation of Angiotensin-(1-7) can be selected from the group consisting of chitosan, sodium hyaluronate, starch (wheat, corn, potato, rice, tapioca), guar gum, Locust bean gum (LBG)/carob, Ceratonia), konjac gum, K, 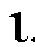 , λ-carrageenan, agarose, sodium alginate, tragacanth, gum arabic/Acacia gummi, pectin (low or high methoxylated) (apple, citrus peel, beet), xanthan gum, gellan gum, dextran, pullulan.

Suitable cellulose derivatives used in microencapsulation of Angiotensin-(1-7) can be selected from the group consisting of methylcellulose (MC), carboxymethylcellulose sodium (CMC-Na), hydroxypropylcellulose (HPC), hydroxypropyl-methylcellulose (HPMC), ethylcellulose, cellulose acetate butyrate or mixtures of at least two components thereof.

Suitable synthetic polymer derivatives used in microencapsulation of Angiotensin-(1-7) can be selected from the group consisting of Poly(lactic acid) (PLA), Polylactic acid-glycolic acid copolymer (PLGA), Polyacrylic acid (Carbopol), Polymethacrylates, Poly-(N-isopropylacrylamide), Polyethylene glycols, Fumaryl diketopiperazine (FDKP) or mixtures of at least two components thereof.

Suitable polymer combinations used in microencapsulation of Angiotensin-(1-7) can be selected from the group consisting of Chitosan-alginate, Agarose-alginate (CaCh), Gum Arabic-Cellulose acetate butyrate (CAB), Gelatin + gum Arabic, Maltodextrin + gum Arabic, CMC-Na + xanthan gum, LBG + PVA, Chitosan + pectin, Gelatin + gum Arabic, Gelatin + chitosan, whey Protein + maltodextrin, Whey protein + alginate, Alginate + gelatin, Alginate + zein, Chitosan+zein, Poly (L-ornithine) +alginate + PLA, PLGA Poly (L-ornithine) +ursodeoxycholic acid, Polystyrene sulfonate, polyallylamine, Poly (ethylene glycol) (PEG)-anthracene alginate, Vinyl-sulfone terminated PEG + alginate, Alginate + Poly-ε-caprolactone, Polypropylene + PMMA + ethylcellulose, PLGA-alginate.

In a further preferred embodiment of the use the formulation can comprise crystalline Angiotensin-(1-7) polymorphs. Surprisingly it was found that Angiotensin-(1-7) can also be administered and is very effective and bioavailable also in a crystalline form. Different polymorphs of the Angiotensin-(1-7) are obtainable, wherein the crystallinity originates from a defined crystal structure of the Angiotensin-(1-7). The degree of crystallinity and the regular arrangement of the Angiotensin-(1-7) in the solid state may for instance be detected within a powder X-ray diffraction (PXRD) experiment.

Within a further preferred aspect of the use the formulation can comprise crystalline Angiotensin-(1-7) solvates. Surprisingly it was found, that Angiotensin-(1-7) including one or more further solvent molecules in a defined and ordered arrangement can also be administered and is very effective and bioavailable in a crystalline form. Different ordered solvates of the Angiotensin-(1-7) are obtainable, wherein the crystallinity originates from a defined crystal structure of the Angiotensin-(1-7) and the solvent molecules. The degree of crystallinity and the regular arrangement of the Angiotensin-(1-7) solvates in the solid state may for instance be detected within a PXRD experiment.

In a preferred aspect of the use the formulation can comprise Angiotensin-(1-7) salts. Besides the use of Angiotensin-(1-7) in the treatment of (SARS)-CoV- or (SARS)-CoV-2-infections as such it is also possible and beneficial to use Angiotensin-(1-7) in a charged form, also comprising one or more counter ions. The Angiotensin-(1-7) may either be positively or negatively charged. Based on the use of a salt form the bioavailability, stability and effectivity of the treatment can be affected.

Within a further preferred characteristic of the use the formulation can comprise freeze dried Angiotensin-(1-7). A further preferred use may include the administration of Angiotensin-(1-7) in a freeze-dried form. Freeze drying may further increase the storage stability of the Angiotensin-(1-7) and enable clean and biocompatible formulations. The Angiotensin-(1-7) may either be freeze dried as such or including a pharmaceutically acceptable carrier material.

It is further within the scope of the current invention to disclose a pharmaceutical composition comprising Angiotensin-(1-7) in a pharmaceutical acceptable carrier. Angiotensin-(1-7) can advantageously be incorporated within a pharmaceutical composition comprising a pharmaceutical acceptable carrier, wherein the overall formulation can be selected from the above-mentioned group of pharmaceutical formulations. Within this group an efficient and storage stable pharmaceutical composition is achieved, wherein the Angiotensin-(1-7) can be delivered spot specific or systemically to a COVID-patient, especially a COVID-19-patient. The composition is able to reduce the effects of the overall infection. The pharmaceutical composition is adaptable and can be used in stages of a (SARS)-CoV- or (SARS)-CoV-2-infection, starting from the early onset up to the late stages, wherein high virus loads are detectable. The pharmaceutical composition is especially helpful is severe cases, wherein patients are hospitalized and receive either non-invasive or invasive ventilation.

As a function of the severity of the (SARS)-CoV- or (SARS)-CoV-2-infection the pharmaceutical composition may provide a dose of 0.05 mcg up to 100 mcg Angiotensin-(1-7) per unit per application. It is further preferred, that a single dose comprises larger than 5 mcg and less or equal than 75 mcg Angiotensin-(1-7) per dose. Furthermore, a single dose may preferably comprise larger than 10 mcg and less or equal than 50 mcg Angiotensin-(1-7) per dose.

As a function of the severity of the (SARS)-CoV- or (SARS)-CoV-2-infection the pharmaceutical composition may provide a dose of 0.05 mcg up to 100 mcg Angiotensin-(1-7) per day. It is further preferred, that a daily dose comprises larger than 1 mcg and less or equal than 85 mcg Angiotensin-(1-7). Furthermore, a daily dose may preferably comprise larger than 15 mcg and less or equal than 65 mcg Angiotensin-(1-7).

In a preferred aspect of the pharmaceutical composition the pharmaceutical composition can comprise crystalline Angiotensin-(1-7). Pharmaceutical compositions comprising Angiotensin-(1-7) in a crystalline from may for instance show a better bioavailability compared to amorphous or unordered Angiotensin-(1-7). For instance, one or more Angiotensin-(1-7) polymorphs can be used within the pharmaceutical compositions at the same time.

In another preferred aspect of the pharmaceutical composition the pharmaceutical composition can comprise salt-like Angiotensin-(1-7). Besides using pharmaceutically acceptable Angiotensin-(1-7) compositions in the treatment of (SARS)-CoV- or (SARS)-CoV-2-infections it is also advantageous and beneficial to incorporate Angiotensin-(1-7) in the pharmaceutical composition in a charged form. The Angiotensin-(1-7) and the counter-ions within the pharmaceutical compositions may for instance increase bioavailability, stability and effectivity of the treatment.

Within a preferred embodiment of the pharmaceutical composition the pharmaceutical composition can comprise encapsulated Angiotensin-(1-7). Angiotensin-(1-7) in an encapsulated form is very suitable in the treatment of COV- and especially (SARS)-CoV- or (SARS)-CoV-2 infections. In an encapsulated form the bioavailability and the release profile can be tailored, resulting in a flexible pharmaceutical composition, which can be adapted for instance with respect to the Angiotensin-(1-7) load and release over time. Suitable encapsulation systems are inter alia mentioned above.

In another preferred aspect the pharmaceutical composition can comprise nanoparticulate Angiotensin-(1-7). Angiotensin-(1-7) in a nano-encapsulated form is very suitable in the treatment of CoV- and especially (SARS)-CoV- or (SARS)-CoV-2-infections. In a nano-encapsulated form the bioavailability and the release profile can be tailored, resulting in a flexible pharmaceutical composition, which can be adapted for instance with respect to the Angiotensin-(1-7) load and release over time. Suitable nano-encapsulation systems are inter alia mentioned above.

## Claims

1. Pharmaceutical composition comprising Angiotensin-(1-7) (Asp-Arg-Val-Tyr-Ile-His-Pro) for use in the treatment of coronavirus infection related diseases, **characterized in that** Angiotensin-(1-7) is administered in a formulation selected from the group consisting of intramuscular, transdermal, pulmonary, lingual, buccal, nasal or a combination of at least two members of said list.

2. Use according to claim 1, wherein the formulation is a transdermal formulation and the transdermal formulation comprises Angiotensin-(1-7) in a form selected from the group consisting of oil-in-water, water-in-oil, gel, solid-lipid nanoparticles (SLN), nanostructured lipid carriers (NLC), microemulsions, transfersomes or a combination of at least two members of said list.

3. Use according to claim 1, wherein the formulation is a pulmonary formulation and the pulmonary formulation comprises Angiotensin-(1-7) in a form selected from the group consisting of liposomal encapsulated, liquid suspension, dry powder or a combination of at least two members of said list.

4. Use according to claim 1, wherein the formulation is a lingual/buccal formulation and the lingual/buccal formulation comprises Angiotensin-(1-7) in a form selected from the group consisting of chewing gum, sublingual, dry powder or a combination of at least two members of said list.

5. Use according to claim 1, wherein the formulation is a nasal formulation and the nasal formulation comprises Angiotensin-(1-7) in a form selected from the group consisting of nanoparticles, oil-in-water, water-in-oil, gel, hyaluronic acid based aqueous solution, DMSO-based solution, dry powder or a combination of at least two members of said list.

6. Use according to any one of the preceding claims, wherein the formulation comprises Angiotensin-(1-7) in the form of an encapsulated Angiotensin-(1-7) particle.

7. Use according to any one of the claims 1-5, wherein the formulation comprises crystalline Angiotensin-(1-7) polymorphs.

8. Use according to any one of the claims 1-5, wherein the formulation comprises crystalline Angiotensin-(1-7) solvates.

9. Use according to any one of the claims 1-5, wherein the formulation comprises Angiotensin-(1-7) salts.

10. Use according to any one of the claims 1-5, wherein the formulation comprises freeze dried Angiotensin-(1-7).

11. Pharmaceutical composition comprising Angiotensin-(1-7) in a pharmaceutical acceptable carrier.

12. Pharmaceutical composition according to claim 11, wherein the pharmaceutical composition comprises crystalline Angiotensin-(1-7).

13. Pharmaceutical composition according to claim 11, wherein the pharmaceutical composition comprises salt-like Angiotensin-(1-7).

14. Pharmaceutical composition according to claim 11, wherein the pharmaceutical composition comprises encapsulated Angiotensin-(1-7).

15. Pharmaceutical composition according to claim 11, wherein the pharmaceutical composition comprises nanoparticulate Angiotensin-(1-7).
